Europäisches Patentamt

European Patent Office

Office européen des brevets

(11) Publication number: **0 151 708
B1**

## EUROPEAN PATENT SPECIFICATION

(45) Date of publication of patent specification: **13.07.88**

(51) Int. Cl.⁴: $C\ 12\ N\ 15/00$

(21) Application number: **84114226.8**

(22) Date of filing: **24.11.84**

(54) **Method for preparing protoplasts of laver thallus.**

(30) Priority: **09.02.84 JP 22415/84**

(43) Date of publication of application:
**21.08.85 Bulletin 85/34**

(45) Publication of the grant of the patent:
**13.07.88 Bulletin 88/28**

(84) Designated Contracting States:
**AT BE CH DE FR GB IT LI NL SE**

(56) References cited:
**NL-A-7 101 939**

**CHEMICAL ABSTRACTS, vol. 86, no. 7, 14th
February 1977, page 226, no. 39777j,
Columbus, Ohio, US; J.D.CLEMENT-METRAL:
"Preparation and some properties of
protoplasts from the red alga Porphyridium
cruentum"**

**AGRIC. BIOL. CHEM., vol. 45, no. 8, 1981, pages
1905-1909; T.YAMADA et al.: "Protoplast
induction in Chlorella species"**

(73) Proprietor: **KOASA TRADING CO.,LTD.
1-7 Meieki 5-chome Nakamura-Ku
Nagoya Aichi-Ken (JP)**

(72) Inventor: **Shibata, Teruhiko
22-15, Karatodai 4-chome
Kita-ku Kohbe Hyogo-ken (JP)**

(74) Representative: **Kraus, Walter, Dr. et al
Patentanwälte Kraus, Weisert & Partner
Thomas-Wimmer-Ring 15
D-8000 München 22 (DE)**

(56) References cited:
**CHEMICAL ABSTRACTS, vol. 71, no. 17, 27th
October 1969, page 27, no. 77477j, Columbus,
Ohio, US; B.D.VANCE et al.: "Preparation of
metabolically active protoplasts of blue-green
algae"**

# 0 151 708

**Description**

Background of the invention

This invention relates to a method for preparing protoplasts of laver thallus which can be effectively used in cell fusion.

Description of the prior art

In recent years, very extensive studies on cell fusion have been being carried on and, with terrestrial plants, the stage of practical application has already been attained in some instances.

However, research reports on the cell fusion of marine algae, especially of *Porphyra* species, are very few and no successful case has been reported as yet.

Basically, the preparation of protoplasts of laver thallus has been considered to be difficult because, in contrast to terrestrial plants in which most of the polysaccharide constituting the cell wall is cellulose, thalli of laver have a cell wall composed principally of hardly digestible polysaccharides including xylan and mannan.

At present, the only attempt to prepare protoplasts from *Porphyra* species is found in the report of Fujita et al., entitled "Separation of protoplasts of laver or green laver by enzyme treatment and their development" [Synopses of Lectures in the Autumn of 1982 (edited by the Japan Fisheries Society), p. 23, 213]. According to the method of Fujita et al., protoplasts are prepared by cutting a thallus of laver into pieces by physical means and then treated these pieces for about 4 hours with a crude enzyme solution obtained from a culture of a strain (strain P-1) of *Pseudomonas* species. However, this method requires a long period of time for the enzyme treatment in the preparation of protoplasts and, moreover, has a high possibility of producing unsound protoplasts and hence interfering with their cell fusion because a thallus of laver having been cut by physical means is treated with enzymes. In cell fusion techniques, the soundness of protoplasts used therefor is presumably a very important factor and the use of unsound protoplasts in cell fusion is considered to give a low rate of fusion and also hinder the growth of the resulting homokaryons in subsequent culture.

Moreover, the reason why a thallus of laver is cut into pieces in the above-described method seems to be based on the recognition that the enzymes yielded by the strain of *Pseudomonas* species do not act on the superficial part of laver but on its cut ends to lyse the cell wall of laver and produce protoplasts.

According to a report of Preston et al., the superficial layer of laver includes mannan distributed in the form of granules, its cell wall is composed of xylan having the form of microfibrils, and porphyran is present as an intercellular substance. Moreover, it has also been reported by L. A. Hanic that the superficial layer includes a thin coat composed of protein. Thus, in view of the above-described histology of laver, it has probably been thought that the preparation of protoplasts from a thallus of laver is impossible unless it is cut into pieces.

The present inventor has investigated the preparation of laver protoplasts on the basis of the idea that, in order to prepare sound protoplasts of laver thallus suitable for use in cell fusion, it is necessary to disintegrate a thallus of laver from its superficial layer without cutting it by physical means, and has found that protoplasts of laver thallus can be obtained in a sound state by treating a thallus of laver with an enzyme solution containing a mannan hydrolase and a xylan hydrolase and, in certain cases, further containing a porphyran hydrolase.

Summary of the invention

As a result of further studies on the preparation of protoplasts of laver thallus, the present inventor has found that sound protoplasts of laver thallus can be prepared in a very short period of time by preliminarily treating a thallus of laver with a protease and then treating it with a combination of $\beta$-1,3-xylanase and $\beta$-1,4-mannanase or a combination of $\beta$-1,3-xylanase, $\beta$-1,4-mannanase and porphyranase, and has thereby completed this invention.

Accordingly, it is the primary object of the present invention to provide a method for preparing protoplasts of laver thallus in a very short period of time in which the resulting protoplasts are sound and can be effectively used in cell fusion.

Other objects of the present invention will be apparent from the following description.

Detailed description of the invention

The characteristic feature of the present invention lies in the fact that sound protoplasts having no damage to the cell membrane can be prepared in a very short period of time by preliminarily treating a thallus of laver with a protease to hydrolyze a portion of the protein present in the superficial layer of its cell wall and then treating the thallus of laver with a combination of $\beta$-1,3-xylanase and $\beta$-1,4-mannanase in the case of a young thallus, or with a combination of the aforesaid enzymes and porphyranase in the case of an adult thallus.

It is to be understood that the method of the present invention is also applicable to the preparation of protoplasts from the *Conchocelis* phase of *Porphyra* species and, therefore, the term "thallus of laver" as used herein also comprehends filaments of the *Conchocelis* phase of *Porphyra* species.

The present inventor isolated and purified the xylan, mannan and porphyran constituting thalli of laver,

2

**0 151 708**

analyzed their structures by the periodate oxidation method, and determined their monosaccharide compositions by subjecting their hydrolyzates to liquid chromatography. As a result, it was that the xylan is a monopolymer of xylose having β-1,3-linkages, the mannan is a monopolymer of mannose having β-1,4-linkages, and the porphyran is a polymer of galactose.

Furthermore, it was also found that, among thalli of laver, young thalli (1—2 mm) are very low in porphyran content, while adult thalli (5 mm or thereabouts) are considerably high in porphyran content.

In this connection, some terrestrial plants rarely include, in addition to cellulose a small amount of xylan as a constituent polysaccharide of the cell wall, but most if it has β-1,4-linkages. On the other hand, in plants (such as devil's-tongue) having mannan as the principal constituent, its monosaccharide components are glucose and mannose joined by β-1,4-linkages.

Thus, on the basis of the above-described findings, the present invention makes it possible to prepare sound protoplasts of laver thallus in a short period of time by preliminarily treating a thallus of laver with a protease to hydrolyze a portion of the protein present in the superficial layer of its cell wall and then treating the thallus of laver with a combination of β-1,3-xylanase and β-1,4-mannanase (in the case of a young thallus) or a combination of these two enzymes and porphyranase (in the case of an adult thallus or the *Conchocelis* phase).

It should be mentioned in this connection that xylan hydrolases are classified into β-1,3-xylanase and B-1,4-xylanase and mannan hydrolases are classified into B-1,3-mannanase and B-1,4-mannanase, and that the treatment of a thallus of laver with a combination of B-1,4-xylanase and B-1,3-mannanase cannot cause hydrolysis of the polysaccharides constituting its cell wall.

As described above, the method of the present invention includes the step of preliminarily treating a thallus of laver with a protease to hydrolyze a portion of the protein present in the superficial layer of its cell wall. Thus is due to the fact that, if a thallus of laver is directly treated with a combination of β-1,3-xylanase and β-1,4-mannanase, the thallus of laver will be gradually disintegrated from its marginal portions as if individual cells were separated one by one, and sound protoplasts cannot always be obtained because their cell membrane itself may suffer damage and, in the worst case, allow the cell contents to flow out. More specifically, when a thallus of laver is preliminarily treated with a protease to hydrolyze the protein present in the superficial layer of its cell wall and then treated with a combination of β-1,3-xylanase and β-1,4-mannanase, the thallus of laver is disintegrated not from its marginal portions but over its whole body, so that the thallus is broken up and its cell wall is ysed in a short period of time (about 30 minutes) to produce protoplasts.

The treatment of the thallus of laver with the protease is preferably carried out at room temperature for a period of about 10 minutes. It is also preferable to use papain as the protease.

The thallus which has been treated with the protease is washed and then treated at room temperature for a period of about 20 minutes with a mixed enzyme solution containing β-1,3-xylanase and β-1,4-mannanase in the case of a young thallus, or treated in the same manner with a mixed enzyme solution containing the aforesaid two enzymes and porphyranase in the case of an adult thallus or the *Conchocelis* phase.

The enzyme solution used for this purpose can be a mixture of crude enzyme solutions containing β-1,3-xylanase and β-1,4-mannanase and, if necessary, further containing porphyranase. Such enzyme solutions may be obtained, for example, by isolating a microorganism of the genus *Pseudomonas* having the ability to hydrolyze hardly digestible polysaccharides, culturing it in a medium containing laver or a laver-derived polysaccharide as an inducer, and removing solid matter from the resulting culture. However, in order to prepare protoplasts in a short period of time, it is preferable to use a mixture of solutions containing a purified preparation of each enzyme.

It has been observed that, when the protoplasts of laver thallus prepared as above in accordance with the present invention are placed in a Petri dish and suspended in artificial sea water (Asp. 12 for algae) having the composition given below, they become attached to the bottom of the Petri dish and grow in a very good condition.

3

Composition of artificial sea water (Asp. 12)

| | |
|---|---|
| NaCl | 28 g |
| $MgSO_4 \cdot 7H_2O$ | 7 g |
| $MgCl_2 \cdot 6H_2O$ | 4 g |
| KCl | 700 mg |
| $CaCl_2 \cdot 2H_2O$ | 1.47 g |
| $NaNO_3$ | 100 mg |
| $K_2HPO_4$ | 10 mg |
| Sodium glycerophosphate | 10 mg |
| Vitamin $B_{12}$ | 0.2 µg |
| Biotin | 1 µg |
| Thiamine | 100 µg |
| P II Metal | 10 ml |
| S II Metal | 10 ml |
| Trisaminomethane | 1 g |
| Distilled water | 1,000 ml |
| pH | 8.0—8.1 |

| Composition of P II metal | | Composition of S II metal | |
|---|---|---|---|
| EDTA | 1 mg | NaBr | 1.2 mg |
| $H_3BO_3$ | 1 mg | $AlCl_3 \cdot 6H_2O$ | 1.2 mg |
| $MnCl_2 \cdot 4H_2O$ | 0.14 mg | $SrCl_2 \cdot 6H_2O$ | 0.6 mg |
| $FeCl_2 \cdot 6H_2O$ | 0.05 mg | $NaMoO_4 \cdot 2H_2O$ | 0.12 mg |
| $ZnCl_2$ | 0.01 mg | PbCl | 0.03 mg |
| $CoCl_2 \cdot 6H_2O$ | 4 µg | KI | 1.5 µg |
| $CuSO_4 \cdot 5H_2O$ | 0.5 µg | Distilled water | 1 ml |
| Distilled water | 1 ml | | |

As described above, the method of the present invention makes it possible to prepare sound protoplasts of laver thallus suitable for purposes of cell fusion in a very short period of time, and is believed to serve for further promotion of cell fusion technology for laver thallus.

To further illustrate the present invention, the following examples are given. However, these examples are intended to be illustrative only and are not to be construed to limit the scope of the invention.

Example 1

About 0.1 g (wet weight) of young thalli (1—2 mm in length) of laver were placed in an L-shaped test tube. After the addition of 10 ml of a 0.5% papain solution (in M/15 Tris-HCl buffer, pH 7.4), the test tube was incubated, with shaking (at 100 strokes per minute), at a temperature of 25°C for a period of 10 minutes. The thalli so treated were thoroughly washed with sea water and then with M/15 phosphate buffer (pH 7.0), and transferred to another L-shaped test tube. After the addition of 5 ml of a β-1,3-xylanase solution and 5 ml of

4

a β-1,4-mannanase solution, the test tube was incubated, with shaking (at 100 strokes per minute), at a temperature of 25°C for a period of 20 minutes.

When the resulting fluid was examined under the microscope, it was confirmed that there were obtained $5.9 \times 10^4$ protoplasts.

The β-1,3-xylanase and β-1,4-mannanase solutions used in this example were prepared in the following manner.

β-1,3-xylanase solution

Among the bacteria attached to thalli of laver being cultivated, ones having the ability to hydrolyze agar were isolated and cultured in a medium comprising sea water to which peptone, yeast extract and the like were added and, moreover, β-1,3-xylan was added as an inducer so as to give a concentration of 1%. The resulting culture was centrifuged to collect the supernatant, which was purified with ammonium sulfate and then dialyzed against M/15 phosphate buffer (pH 7.0). The β-1,3-xylanase solution thus obtained had an activity of 23 units/ml.

β-1,4-mannanase solution

The same procedure as described above was repeated except that there was used the aforesaid medium to which, in place of the β-1,3-xylan, β-1,4-mannan was added as the inducer so as to give a concentration of 1%.

The β-1,4-mannanase solution thus obtained had an activity of 18 units/ml.

The papain used was a product (10 units/mg) of Wako Junyaku Kogyo K.K.

Then, the protoplasts of laver thallus obtained as above were cultured according to the following procedure.

Using artificial sea water Asp. 12 as the medium, $8 \times 10^4$ protoplasts were cultured at a temperature of 17°C under illumination at a light intensity of 5,000 lux (with alternation of 9-hour light periods and 15-hour dark periods). The results of observations made under the microscope in the course of the culture are given in the following table.

| | After 3 days of culture | After 4 days of culture | After 5 days of culture |
|---|---|---|---|
| Findings | Formation of cell wall in 10—20% | Formation of cell wall in 60—70% | Formation of cell wall and cell division in 60—70% |

Example 2

Protoplasts were prepared according to the same procedure as described in Example 1, except that an adult thallus of laver (about 5 cm in length) was used in place of the young thalli and 5 ml of a porphyranase solution was used in addition to the β-1,3-xylanase solution (5 ml) and the β-1,4-mannanase solution (5 ml).

When the resulting fluid was examined under the microscope, it was confirmed that there were obtained $4.0 \times 10^4$ protoplasts.

The porphyranase solution used in this example was prepared in the same manner as described above for the β-1,3-xylanase solution, except that, in place of the β-1,3-xylan, porphyran was added to the medium so as to give a concentration of 1%.

**Claims**

1. A method for preparing protoplasts of laver thallus suitable for use in cell fusion which comprises treating a thallus of laver with a protease and then treating said thallus of laver with a combination of β-1,3-xylanase and β-1,4-mannanase.

2. A method for preparing protoplasts of laver thallus suitable for use in cell fusion which comprises treating a thallus of laver with a protease and then treating said thallus of laver with a combination of β-1,3-xylanase, β-1,4-mannanase and porphyranase.

3. A method as claimed in claim 1 wherein said protease is papain.

4. A method as claimed in claim 2 wherein said protease is papain.

5. A method as claimed in claim 1 wherein said thallus of laver is a young thallus.

6. A method as claimed in claim 2 wherein said thallus of laver is an adult thallus or the *Conchocelis* phase of *Porphyre* species.

7. A method as claimed in claim 1 wherein said thallus of laver is treated with a mixed enzyme solution containing β-1,3-xylanase and β-1,4-mannanase at room temperature for a period of about 20 minutes.

8. A method as claimed in claim 2 wherein said thallus of laver is treated with a mixed enzyme solution containing β-1,3-xylanase, β-1,4-mannanase and porphyranase at room temperature for a period of about 20 minutes.

9. A method as claimed in claim 3 wherein the treatment with papain is carried out at room temperature for a period of about 10 minutes.

10. A method as claimed in claim 4 wherein the treatment with papain is carried out at room temperature for a period of about 10 minutes.


**Patentansprüche**

1. Verfahren zur Herstellung von Seelattich-(bzw. Purpurtang)-Thallusprotoplasten, die für die Verwendung bei einer Zellfusion geeignet sind, dadurch gekennzeichnet, daß man den Thallus von Seelattich (bzw. Purpurtang) mit einer Protease behandelt und dann den Seelattich-(bzw. Purpurtang)-Thallus mit einem Gemisch aus β-1,3-Xylanase und β-1,4-Mannanase behandelt.

2. Verfahren zur Herstellung von Seelattich-(bzw. Purpurtang)-Thallusprotoplasten, die für die Verwendung bei einer Zellfusion geeignet sind, dadurch gekennzeichnet, daß man den Thallus von Seelattich (bzw. Purpurtang) mit einer Protease behandelt und dann den Seelattich-(bzw. Purpurtang)-Thallus mit einem Gemisch aus β-1,3-Xylanase, β-1,4-Mannanase und Prophyranase behandelt.

3. Verfahren nach Anspruch 1, dadurch gekennzeichnet, daß die Protease Papain ist.

4. Verfahren nach Anspruch 2, dadurch gekennzeichnet, daß die Protease Papain ist.

5. Verfahren nach Anspruch 1, dadurch gekennzeichnet, daß der Thallus von Seelattich (bzw. Purpurtang) ein junger Thallus ist.

6. Verfahren nach Anspruch 2, dadurch gekennzeichnet, daß der Thallus von Seelattich (bzw. Purpurtang) ein ausgewachsener Thallus oder die *Conchocelis*-Phase von *Porphyre*-Spezies ist.

7. Verfahren nach Anspruch 1, dadurch gekennzeichnet, daß der Seelattich-(bzw. Purpurtang)-Thallus mit einer Lösung einer Enzymmischung, welche β-1,3-Xylanase und β-1,4-Mannanase enthält, bei Raumtemperatur während einer Zeit von etwa 20 Minuten behandelt wird.

8. Verfahren nach Anspruch 2, dadurch gekennzeichnet, daß der Seelattich-(bzw. Purpurtang)-Thallus mit einer Lösung einer Enzymmisch, welche β-1,3-Xylanase und β-1,4-Mannanase enthält, bei Raumtemperatur während einer Zeit von etwa 20 Minuten behandelt wird.

9. Verfahren nach Anspruch 3, dadurch gekennzeichnet, daß die Papain-Behandlung bei Raumtemperatur während einer Zeit von etwa 10 Minuten durchgeführt wird.

10. Verfahren nach Anspruch 4, dadurch gekennzeichnet, daß die Papain-Behandlung bei Raumtemperatur während einer Zeit von etwa 10 Minuten durchgeführt wird.


**Revendications**

1. Procédé pour préparer des protoplastes de tholle de laitue de mer utiles dans la fusion cellulaire, caractérisé en ce qu'on traite un thalle de laitue de mer avec une protéase et que l'on traite ensuite ce thalle de laitue de mer avec une combinaison de β-1,3-xylanase et de β-1,4-mannanase.

2. Procédé pour préparer des protoplastes de thalle de laitue de mer utiles dans la fusion cellulaire, caractérisé en ce qu'on traite un thalle de laitue de mer avec une protéase et que l'on traite ensuite ce thalle de laitue de mer avec une combinaison de β-1,3-xylanase, de β-1,4-mannanase et de porphyranase.

3. Procédé suivant la revendication 1, caractérisé en ce que cette protéase est la papaïne.

4. Procédé suivant la revendication 2, caractérisé en ce que cette protéase est la papaïne.

5. Procédé suivant la revendication 1, caractérisé en ce que ce thalle de laitue de mer est un thalle jeune.

6. Procédé suivant la revendication 2, caractérisé en ce que ce thalle de laitue de mer est un thalle adulte ou la phase *Conchocelis* de l'espèce *Porphyre*.

7. Procédé suivant la revendication 1, caractérisé en ce que ce thalle de laitue de mer est traité avec une solution d'enzymes mélangées contenant de la β-1,3-xylanase et de la β-1,4-mannanase à la température ambiante pendant une période d'environ 20 minutes.

8. Procédé suivant la revendication 2, caractérisé en ce que ce thalle de laitue de mer est traité avec une solution d'enzymes mélangées contenant de la β-1,3-xylanase, de la β-1,4-mannanase et de la porphyranase à la température ambiante pendant une période d'environ 20 minutes.

9. Procédé suivant la revendication 3, caractérisé en ce que le traitement avec la papaïne est effectué à la température ambiante pendant une période d'environ 10 minutes.

10. Procédé suivant la revendication 4, caractérisé en ce que le traitement avec la papaïne est effectué à la température ambiante pendant une période d'environ 10 minutes.